# EUROPEAN PATENT SPECIFICATION

(11) **EP 2 385 993 B1**
(45) Date of publication and mention of the grant of the patent: **30.03.2016**
(21) Application number: 10704239.2
(22) Date of filing: 08.01.2010
(51) Int. Cl.: C12Q 1/68

(54) **GENETIC ANALYSIS OF CELLS**
GENETISCHE ANALYSE VON ZELLEN
ANALYSE GÉNÉTIQUE DE CELLULES

(30) Priority: 09.01.2009 US 143745 P
(43) Date of publication of application: 16.11.2011
(73) Proprietor: Intrexon Corporation, Blacksburg, Virginia 24060 (US)
(72) Inventor: KAMME, Fredrik, San Diego CA 92130 (US); BRIGHT, Gary, San Diego CA 92127 (US); ANGELBORG, Gustaf, San Diego CA 92111 (US); LINTON, James, San Diego CA 92128 (US); KOLLER, Manfred, San Diego CA 92128 (US)
(74) Representative: Stuttard, Garry Philip
(86) International application number: PCT/US2010/020562
(87) International publication number: WO 2010/081052

(56) References cited:
- WO-A1-01/68110
- WO-A1-98/30384
- WO-A2-2007/147079
- US-A- 6 156 576
- US-A1- 2008 166 793
- EL-SAYED ET AL: "Selective laser photo-thermal therapy of epithelial carcinoma using anti-EGFR antibody conjugated gold nanoparticles" CANCER LETTERS, NEW YORK, NY, US LNKD- DOI:10.1016/J.CANLET.2005.07.035, vol. 239, no. 1, 28 July 2006 (2006-07-28), pages 129-135, XP005525665 ISSN: 0304-3835
- Quiagen: "RNeasy Midi / Maxi Handbook Second Edition"[Online] June 2001 (2001-06), pages 1-108, XP002583654 Retrieved from the Internet: URL:http://www.qiagen.com/literature/rende r.aspx?id=162> [retrieved on 2010-05-26]
- DIXON A K A K ET AL: "Gene-expression analysis at the single-cell level" TRENDS IN PHARMACOLOGICAL SCIENCES, ELSEVIER, HAYWARTH, GB LNKD- DOI:10.1016/S0165-6147(99)01433-9, vol. 21, no. 2, 1 February 2000 (2000-02-01), pages 65-70, XP004189124 ISSN: 0165-6147

## Description

### BACKGROUND

The analysis of cellular nucleic acids, such as mRNA or genomic DNA, of specific cells in a heterogeneous mixture of cells is typically performed by either analyzing a few analytes, typically 1-10, in situ, or by mechanically isolating the desired cell(s) from the background population and then collecting and analyzing the contents of the isolated cell(s).

The in situ analysis approaches which directly analyze cells within an unpurified mixture include in situ hybridization (ISH). ISH is generally used to detect specific mRNA or DNA sequences within cells or tissue sections, and is limited by the number of analytes that can be measured in one experiment. Typically, ISH is performed for 1 or 2 analytes in one experiment (Young WS 3rd. Methods Enzymol. (1989) 168:702-10). The in situ analysis approaches are all characterized by direct analysis of the cellular contents in the context of the cell sample with no cell purification and no collection or storage of the cellular contents for subsequent analysis. In situ analysis approaches are generally performed via manual microscopic observation and are not amenable to high-throughput scale up or automation.

The mechanical isolation approaches which purify the cells prior to collection of their cellular contents include antibody-based magnetic bead cell purification (e.g., MACS® Cell Separation, Miltenyi Biotec, Germany), flow cytometric cell sorting, laser microdissection or mechanical microdissection. Once the cell(s) have been isolated, highly multiplexed analyses may be performed, such as complete gene expression profiling. Each of these various cell isolation methods has its own benefits and limitations. For example, magnetic bead purification and flow sorting require a relatively large number of cells to work properly, absolute purity of isolated cells is rarely achieved and selection parameters are generally limited to a few surface markers. Laser microdissection may isolate a single cell, or many cells, but is limited in the number of samples that can be processed and is generally applied to tissue specimens with specific requirements (Luo et al., Nat Med. (1999) 5(1):117-22). Mechanical microdissection is limited in both the number of cells and samples that may be processed. With all of the isolation methods, analyzing cells, particularly rare cells, becomes challenging due to yield issues in which many of the cells of interest are lost during processing.

One application of nucleic acid analysis of specific cells in a complex mixture of different cell types is the genetic analysis of circulating and disseminated tumor cells. Such cells are shed by a primary tumor and exist in the blood or lymph circulation or reside in various tissues. Disseminated tumor cells are believed to be the cause of metastases. Given that the majority of deaths from cancer disease are due to metastases and not the primary tumor, circulating and disseminated tumor cells have become the subject of intense diagnostic interest. For example, Veridex LLC (Warren, NJ) has launched a FDA-approved diagnostic test which enumerates the number of circulating breast tumor cells as a prognostic tool. Apart from the diagnostic value of detecting the presence of circulating tumor cells, however, it is highly desirable to be able to molecularly classify these cells. Such information may reveal better prognostic information and guide treatment options. For example, the Her-2 receptor, which is the target for Herceptin treatment, is only over-expressed in a sub-population of breast cancer patients, leaving the remaining population of breast cancer patients unresponsive to Herceptin treatment. This example highlights the need for personalized cancer treatment. Molecular profiling of circulating and disseminated tumor cells has posed a significant technical challenge, both in the discovery phase where suitable markers are identified, and in the diagnostic phase where identified markers are implemented. The challenge lies in the scarcity of the desired cells, from as infrequently as 1 in 100,000 to 1 in 10,000,000, which requires an exceptionally efficient process to purify.

To date, genetic analysis or molecular profiling of circulating tumor cells has predominantly been performed on tumor cells enriched through antibody-mediated isolation. Smirnov et al., Cancer Res. (2005) 65(12):4993-7 reported on gene expression profiling of circulating tumor cells enriched by immunomagnetic isolation from human blood. A limitation in the reported study is the lack of purity of the enriched tumor cells which were outnumbered by contaminating leukocytes. Useful gene expression data could only be generated if at least 100 tumor cells were obtained and the background was less than 1,000-10,000 leukocytes. These limitations restricted the selection of patients to those who had high circulating tumor cell counts, >100/7.5 ml blood, which is a serious restriction; as a study showed that the majority of cancer patients have less than 10 circulating tumor cells in 7.5 ml blood (Allard et al., Clin Cancer Res. (2004) 10(20):6897-904). Furthermore, contamination with leukocytes limited the analysis to genes that were highly expressed by the tumor cells. Finally, positive selection of circulating tumor cells using an antibody directed against a specific antigen, such as EpCAM, has inherent weaknesses. It is known that circulating tumor cells display significant heterogeneity in the amount of surface antigen exposed, leading to variable efficiency in capturing these cells (Allard et al., Clin Cancer Res. (2004) 10(20):6897-904), which in turn compromises assay sensitivity.

A different system for isolating circulating tumor cells using antibody selection is the CTC chip (Nagrath et al., Nature. (2007) 450(7173): 1235-9), which consists of a flow chamber with 78,000 microposts, manufactured by deep ion etching. The microposts are coated with an antibody for EpCAM. As blood is pumped through the chamber, circulating tumor cells are retained on the microposts. Captured cells have been analyzed by RT-PCR for a small number of genes. Published data show variable purity of isolated circulating tumor cells, with an average purity of 56% across 6 cancer types (Nagrath et al., Nature. (2007) 450(7173):1235-9). Again, sample impurity will compromise the quality of gene expression profiles. Also, this method is susceptible to variability in sensitivity caused by variation in EpCAM amount displayed by the tumor cells.

US-A-6156576 discloses a method for lysing a cell of interest in a population of cells by a laser beam and collecting and analysing the cell content. WO-A-2007/147079 discloses a method for detecting fetal or cancer cells in a mixed sample by splitting the sample into discrete portions and lysing the cells in each portion before extracting RNA from the cell lysate. WO-A-01/68110 discloses the use of an energy beam to lyse a labelled sub-population of cells. El-Sayed et a/: "Selective laser photo-thermal therapy of epithelial carcinoma using anti-EGFR antibody conjugated gold nanoparticles" CANCER LETTERS, NEW YORK, NY, US LNKD-DOI:10.1016/J.CANLET.2005.07.035, vol. 239, no. 1, 28 July 2006 (2006-07-28), pages 129-135, XP005525665 ISSN: 0304-3835 discloses the use of a gold-labelled antibody to target cells of interest and the use of a laser to beam to selectively lyse the target cells.

Without limiting the scope of this disclosure as expressed by the claims which follow, its more prominent features will now be discussed briefly. After considering this discussion, and particularly after reading the section entitled "Detailed Description" one will understand how the features of this technology provide advantages that include relatively rapid and precise analysis of cells in a cell population, including rare cells.
The present invention relates to a method for isolating nucleic acids from rare cells within a heterogeneous population of cells, comprising:
(a) partitioning the heterogeneous population of cells into a plurality of bins, such that the concentration of the rare cells relative to non-rare cells is increased within the bins containing the rare cells by a factor of at least X-fold, where X is the number of bins divided by the number of rare cells in the heterogeneous population of cells, wherein the rare cells are present in the heterogeneous population of cells at a concentration of 1 in 1,000 to 1 in 100,000,000;
(b) imaging the entire area of each bin to determine which bins contain the rare cells;
(c) locating the positions of the rare cells within the bins containing the rare cells;
(d) directing an energy beam to the positions of the rare cells, within the bins containing the rare cells, to cause specific lysis of the rare cells without significant lysis of non-rare cells, and release of the nucleic acids from the rare cells into the medium,
wherein less than 10% of non-rare cells in the heterogeneous population of cells within the bin are lysed; and
(e) collecting the medium containing released nucleic acids from the bin containing the rare cells that have been lysed, resulting in collection of the nucleic acids from the rare cells.

In some embodiments the locating can be performed for example by reference to the images of the bins. In some embodiments the collecting can result in collection of the nucleic acids from the rare cells with up to a Y-fold enrichment of rare cell nucleic acids versus non-rare cell nucleic acids, where Y is the number of unlysed non-rare cells in the bin. In some embodiments Y may be, for example, approximately 10, 30, 100, 300, 1,000, 3,000, 10,000, 30,000, or 100,000.

In some embodiments the methods may further include, for example, contacting the heterogeneous population of cells with an agent that selectively binds to the rare cells, wherein the agent generates a signal detectable as a property of light. Also, in some embodiments, the methods can further include, for example, adding RNAse inhibitor anywhere between steps (a) and (e). In some embodiments, X can be, for example, approximately 10, 30, 100, 300, 1,000, 3,000, 10,000, 30,000, or 100,000. In some embodiments, the bins may be, for example, wells of a multi-well plate.

In some embodiments, the methods described above and elsewhere herein may further include, for example, labeling the rare cells with a nanoparticle label. Also, the nanoparticle labeling can be used to improve the efficiency of the energy beam-mediated cell lysis.

In some embodiments the rare cell may be, for example, without being limited thereto, a nucleated blood cell, a primary cell, a cell line, a tumor cell, a diseased cell, an infected cell, a recombinant cell, a transfected cell, an engineered cells, or a mutated cell. In some embodiments, the cell population may be, for example, without limitation, a cell population from blood, lymph, cerebrospinal fluid, bone marrow, surgical specimens, a biopsy, a cell culture, a cell library, an engineered cell population, and the like. In some embodiments the methods may further include, for example, contacting the population of cells with a label specific to the rare cell. The label may include, for example, one or more of a polyclonal or monoclonal antibody, a fragment of an antibody, a lectin, a ligand, a protein, a peptide, a lipid, an amino acid, a nucleic acid, a modified nucleic acid such as Locked Nucleic Acid (LNA), a synthetic small molecule, or any other moiety for labeling. Without being limited thereto, in some embodiments the label may include, for example, one or more of horseradish peroxidase, alkaline phosphatase, beta-galactosidase, beta-lactamase, Cy3 or Cy5, fluorescein isothiocyanate, phycoallocyanin, phycoerythrin, rhodamine, 6-carboxy-X-rhodamine (ROX), 6-carboxyrhodamine (R6G), Texas Red, ALEXAFluor Dyes, BODIPY fluorophores, Oregon Green, coumarin and coumarin derivatives, or TAMRA. In some embodiments, the label further may include a nanoparticle. In some embodiments, the nanoparticle may improve the efficiency of the energy beam-mediated cell lysis.

In some embodiments the locating can include, for example, imaging the population of cells. Also, the locating can include, for example, locating the rare cell based upon the presence of the label. In some embodiments, the methods further may include providing an RNAse inhibitor. In some embodiments the methods further may include, for example, adding an Fc Receptor-blocking reagent.

In some embodiments the rare cell can be present in the population of cells at a concentration of between about 1 in 100,000 cells and about 1 in 10,000,000 cells, for example.

In some embodiments, the methods further may include, for example, collecting at least said released cell contents. For example, the cell contents may be, without limitation, nucleic acids, polypeptides, proteins, lipids, organelles, etc.

In some embodiments, the method for isolating nucleic acids may further comprise analysing the cell contents, analyzing may include, for example, one or more of PCR, RT-PCR, quantitative RT-PCR, microarray analysis, nuclease protection analysis, Quantigene analysis, Taqman SNP assay, PCR-restriction fragment length polymorphism, RNA sequencing, DNA sequencing, next generation sequencing, Single Molecule Real Time (SMRT) DNA sequencing technology, or Nanostring technology.

The foregoing is a summary and thus contains, by necessity, simplifications, generalizations, and omissions of detail; consequently, those skilled in the art will appreciate that the summary is illustrative only and is not intended to be in any way limiting. Other aspects, features, and advantages of the methods, compositions and/or devices and/or other subject matter described herein will become apparent in the teachings set forth herein. The summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

### BRIEF DESCRIPTION OF THE DRAWINGS

The foregoing and other features of the present disclosure will become more fully apparent from the following description and appended claims, taken in conjunction with the accompanying drawings. Understanding that these drawings depict only several embodiments in accordance with the disclosure and are, therefore, not to be considered limiting of its scope, the disclosure will be described with additional specificity and detail through use of the accompanying drawings.
Figure 1 is a graph depicting RNAse activity in tissue culture wells after different treatments.
Figure 2 is a graph depicting the effect of various media formulations on the yield of RNA from lysed cells.
Figure 3 is an image of an immunostained SW480 cell in a background of human PBMCs. The arrow indicates the SW480 cell.
Figure 4 is a sequence trace showing the presence of a mutation in codon 12 of the Ki-ras gene from a single lysed SW480 cell.
Figure 5 is a graph depicting the impact of gold nanoparticle labeling on laser-mediated lysis efficiency.
Figure 6 is a table showing the detection of five selected genes in 16 single tumor cell samples.

### DETAILED DESCRIPTION

The teachings herein can be applied in a multitude of different ways, including for example, as defined and covered by the claims. It should be apparent that the embodiments herein may be embodied in a wide variety of forms and that any specific structure, function, or both being disclosed herein is merely representative. Based on the teachings herein one skilled in the art should appreciate that an aspect disclosed herein may be implemented independently of any other aspect and that two or more of these aspects may be combined in various ways. For example, a system or apparatus may be implemented or a method may be practiced by one of skill in the art using any reasonable number or combination of the aspects set forth herein. In addition, such a system or apparatus may be implemented or such a method may be practiced using other structure, functionality, or structure and functionality in addition to or other than one or more of the aspects set forth herein. It will be readily understood that the aspects of the present disclosure, as generally described herein, and illustrated in the Figures, can be arranged, substituted, combined, and designed in a wide variety of different configurations, all of which are explicitly contemplated and made part of this disclosure. It is to be understood that the disclosed embodiments are not limited to the examples described below, as other embodiments may fall within disclosure and the claims.

Some embodiments disclosed herein relate to methods for specific genetic analysis of cells present within a heterogeneous population of cells. In some embodiments, cells of interest are identified, the identified cells are caused to release their cellular contents into the surrounding culture medium by irradiating said cells with sufficient energy to cause the lysis of the targeted cell or cells, culture medium containing released nucleic acids is sampled and materials in the sampled culture medium, such as for example, nucleic acids present in the sampled culture medium are analyzed. In some preferred embodiments, a rare cell is present in a given population of cells as infrequently as between 1 in 100,000 to 1 in 1,000,000. In other preferred embodiments, a rare cell is present in a given population of cells as infrequently as between 1 in 1,000,000 to 1 in 10,000,000.

A unique property of the method for the analysis of rare cells is that the cell to be analyzed need not be purified or mechanically manipulated in any manner prior to analysis. Furthermore, the methods can be very specific. The specificity of the analysis lies in the precision of the irradiation of the cell to be lysed. Using a laser, for example, single cells can be targeted and specifically lysed within a mixed population of cells, enabling extremely high specificity in cell analysis. In addition, specific identification of cells of interest may be used for detection purposes only, for example, rather than for mechanical isolation. This attribute can allow for greater tolerances in terms of intensity of cell labeling as no mechanical constraints need to be considered. Also, a combination of different labels can easily be used in the current technology. Finally, functional labels, such as measurement of secreted products (Hanania et al, Biotech. and Bioengineering, 91(7), 2005), can be employed for circulating tumor cell identification in some embodiments. Secreted products may be captured on the surface of the cell or in the vicinity of the cell to be analyzed and subsequently detected, for example, using labeled antibodies as described in U.S. Patent No. 7,425,426.

As mentioned above, the methods can be utilized to analyze materials from cells such as for example, nucleic acids from cells of interest present in a mixture of other cells. Nucleic acids are polymers of ribonucleotides or deoxyribonucleotides, or a mixture of both. RNA is a polymer of ribonucleotides, typically 50-10,000 nucleotides long and DNA is a polymer of deoxyribonucleotides, typically 50-220,000,000 nucleotides long. Without being limited thereto, the nucleic acids that can be obtained from lysed cells using this technology include messenger RNA (mRNA), micro RNA, tRNA, rRNA, viral RNA, RNA expressed from an inserted construct, such as short hairpin RNA (shRNA), or RNA transfected into cells, such as siRNA, genomic DNA, mitochondrial DNA and viral DNA.

The obtained nucleic acids may be analyzed in a variety of ways including without limitation PCR, RT-PCR, quantitative RT-PCR using Taqman probes or Sybr Green chemistry, quantitative RT-PCR using for example the Mass Array system from Sequenom (San Diego, CA), microarray analysis, nuclease protection analysis, Quantigene analysis (Panomics), Taqman SNP assay, PCR-restriction fragment length polymorphism, RNA sequencing, DNA sequencing, next generation sequencing using the Illumina Genome Analyzer or the ABI Solid instrument or the Roche 454 instrument or the Heliscope instrument from Helicos Biosciences Corporation (Cambridge, MA), Single Molecule Real Time (SMRT) DNA sequencing technology (Pacific Biosciences, Menlo Park, CA) and Nanostring technology (Nanostring Technologies, Seattle, WA).

Cells can be of any origin, including prokaryotic and eukaryotic cells. Non-limiting examples include mammalian cells, rodent cells, non-human primate cells and human cells. Cells may be obtained, for example, from blood, lymph, cerebrospinal fluid, bone marrow, surgical specimens, biopsies, or the like. Cells to be analyzed also may be obtained from, for example, cell cultures, cell libraries and engineered cell populations. Prior to use in the methods disclosed herein, cells may be purified, for example, by density centrifugation, immunomagnetic enrichment, enrichment through immobilized antibody capture, fluorescence activated cell sorting (FACS), membrane filtration, agglutination of irrelevant cells, and chemical lysis of irrelevant cells. Cells can include, for example, nucleated blood cells, primary cells, cell lines, tumor cells, diseased cells, infected cells, transfected cells, engineered cells, mutated cells, and the like.

Release of cellular contents from targeted cells may be achieved by irradiating the targeted cells with a dose of radiation sufficient to partially cause lysis of the cells. A preferred source of radiation is a laser of a wavelength and energy sufficient to cause cell lysis. In some embodiments, lasers useful in the methods of the present disclosure are lasers that deliver radiation having a wavelength equal to or between any range selected from the group of 100, 200, 300, 400, 500, 600, 700, 800, 900, 1000, 1200, 1400, 1600, 1800, 2000, 2200, 2400, 2600, 2800 and 3000 nm. In some embodiments, for example, the wavelength can be between about 355 nm and about 2940 nm, preferably 355 and 1064 nm, most preferably 355 and 532 nm. In some embodiments, lasers useful in the methods of the present disclosure are capable of delivering a dose of radiation having an energy density selected from the group of less than, greater than, equal to or any number in between 0.1, 0.2, 0.3, 0.4, 0.5, 0.6, 0.7, 0.8, 0.9, 1, 2, 3, 4, 5, 6, 7, 8, 9, 10, 20, 30, 30, 40, 50, 60, 70, 80, 90, and 100 J/cm². In some embodiments, lasers useful in the methods of the present disclosure are capable of delivering a dose of radiation having an irradiance selected from the group of less than, greater than, equal to, or any number in between 10⁷, 10⁸, 10⁹, 10¹⁰ and 10¹¹ W/cm². As used herein, the term irradiance means power per area, and is often expressed in units of watts per square centimeter. In some embodiments the LEAP™ Cell Processing Workstation (Cyntellect Inc., San Diego, CA) can be used to identify and irradiate cells. Also, the devices, systems and methods disclosed in U.S. Patent No. 6,514,722 may be utilized.

A laser may be used to cause specific lysis of the rare cells without significant lysis of other cells. Without significant lysis of other cells indicates that less than 10% of non-rare cells in a population of cells are lysed by the laser. In some preferred embodiments, without significant lysis of other cells indicates that less than 9%, 8%, 7%, 6%, 5%, 4%, 3% or 2% of non-rare cells in a population of cells are lysed by the laser. In more preferred embodiments, without significant lysis of other cells indicates that less than 1% of non-rare cells in a population of cells are lysed by the laser. In even more preferred embodiments, without significant lysis of other cells indicates that fewer non-rare cells than rare cells are lysed by the laser.

In some embodiments, the methods described herein will image at least, equal to or any number in between 1, 2, 3, 4, 5, 6, 7, 8, 9, 10, 11, 12, 13, 14, 15, 16, 17, 18, 19 and 20 square centimeters of a biological specimen per minute. In some embodiments, the methods described herein are performed on populations of cells comprising at least 0.05, 0.1, 0.25, 0.5, 1, 20, 4 or 8 million cells of a biological specimen per minute.

An RNase inhibitor is a protein, protein fragment, peptide or small molecule which inhibits the activity of any or all of the known RNAses, including RNase A, RNase B, RNase C, RNase T1, RNase H, RNase P, RNAse I and RNAse III. Some examples of known, but non-limiting, RNAse inhibitors include ScriptGuard (Epicentre Biotechnologies, Madison, WI), Superase-in (Ambion, Austin, TX), Stop RNase Inhibitor (5 PRIME Inc, Gaithersburg, MD), ANTI-RNase (Ambion), RNase Inhibitor (Cloned) (Ambion), RNaseOUT™ (Invitrogen, Carlsbad, CA), Ribonuclease Inhib III (Invitrogen), RNasin® (Promega, Madison, WI), Protector RNase Inhibitor (Roche Applied Science, Indianapolis, IN), Placental RNase Inhibitor (USB, Cleveland, OH) and ProtectRNA™ (Sigma, St Louis, MO). In some embodiments, an RNase inhibitor may be added to the location of the cell, for example, a well containing the cell or cells to be analyzed, at a concentration sufficient to significantly inhibit RNAse activity in the well, by 1-100%, preferably 20-100%, most preferably 50-100%.

In some embodiments, identification of a cell or cells of interest may be accomplished using an agent which specifically binds the cell(s) of interest, but not other cells in the mixture, which are not to be analyzed. The labeling agent can be any suitable agent, including for example, a polyclonal or monoclonal antibody, or a fragment thereof such as Fab, F(ab')2, Fd and Fv. The labeling agent can be a lectin, for example. The labeling agent can be labeled using any moiety capable of generating a detectable signal, for example a signal detectable as a property of light, such as fluorescence, chemiluminescence, fluorescence lifetime, fluorescence polarization, diffraction, and the like. The labeling agent can also be labeled using an enzyme marker, such as for example, horseradish peroxidase, alkaline phosphatase, beta-galactosidase, beta-lactamase, and the like. In some aspects, fluorophores are preferred. Suitable fluorophores may include, for example, Cy3 or Cy5 which are preferred, fluorescein isothiocyanate, phycoallocyanin, phycoerythrin, which is preferred, rhodamine, 6-carboxy-X-rhodamine (ROX), 6-carboxyrhodamine (R6G), Texas Red, ALEXAFluor Dyes, BODIPY fluorophores, Oregon Green, coumarin and coumarin derivatives, TAMRA and the like.

In some embodiments an Fc Receptor-blocking reagent also may be utilized. For example, the blocking reagent can prevent cell-mediated killing of targeted or labeled cells. Cell-mediated killing of spiked tumor cells was observed after staining a mix of tumor cells and primary human PBMCs with a primary antibody against a surface antigen on the tumor cells. This effect was evident as the attachment of PBMCs, likely monocytes or macrophages, to tumor cells was clearly observed. This attachment was likely mediated by the antibody used, even though this was a mouse monoclonal antibody. In some aspects, inclusion of an Fc Receptor-blocking reagent are used to prevent this effect, which may be undesired. Fc Receptor-blocking reagents block the interaction of the Fc-region of antibodies with Fc receptors present on cells. An Fc Receptor-blocking reagent may be, for example, an immunoglobulin that competes with the antibody used for cell labeling with respect to receptor binding. Furthermore, specific antibodies that bind to and block Fc receptors may also be used. Small synthetic molecules that inhibit the binding of the Fc region of antibodies to the Fc receptor may be also be used as Fc receptor blocking reagents. Suitable blocking reagents include, for example, the FcR blocking reagent from Miltenyi Biotec (Auburn, CA).

Microarrays used for expression profiling come in several different formats which are known to those skilled in the art. Non-limiting examples are given in U.S. Patent Nos. 5,445,934, 7,378,236 and 6,326,489. Most microarrays use spatially arranged stretches of DNA to measure the amount of corresponding cDNA or cRNA (collectively known as "targets") in the solution by hybridization. The signal generated from a spot on the microarray correlates to the expression of that particular sequence, typically a cellular gene, in the sample. Typically the expression of many hundreds to several thousands of different cDNAs or cRNAs is assayed on a microarray. Microarrays also can be used to assess copy number variation of genes in genomic DNA (Pinkel et al., 1998), presence of single nucleotide polymorphisms (Pastinen et al., 2000) and genomic DNA methylation status (Yan et al., 2001). Novel microarrays include bead-based arrays such as the Veracode system (Illumina, San Diego, CA). Nanostring (Seattle, WA) markets a "reverse" random ordered microarray, where DNA targets are immobilized and analyzed on a planar surface. Any such microarray can be incorporated into the methods, systems and apparatuses disclosed herein.

In some embodiments, nanoparticles may be incorporated into the methods, to aid in the lysis of the cells. Nanoparticles can be used to facilitate laser-mediated lysis of cells. A nanoparticle is a particle with a size range of 1 -1000 nm, preferably 2 - 500 nm, most preferably 3 - 100 nm. Nanoparticles can be made of a wide variety of materials, including precious metals such as gold and silver, and semiconductor materials such as indium phosphide and cadmiuim sulfide. Nanoparticles can be manufactured via different methods known to those skilled in the art. Examples include gold nanoparticles formed via the controlled precipitation of a gold solution (Turkevich at el., 1951). Nanoparticles may be coated to stabilize the particle in suspension or reduce the cell toxicity of the nanoparticle. For example, gold nanoparticles may be coated with bovine serum albumin to stabilize the suspension. Nanoparticles may be attached to molecules to promote their association with cells. Non-limiting examples of suitable molecules for attachment to nanoparticles include antibodies, antibody fragments, proteins, peptides, lectins, lipids, amino acids, nucleic acids, modified nucleic acids such as Locked Nucleic Acid (LNA) and synthetic small molecules. Some non-limiting methods for attaching said molecules to nanoparticles are known to those skilled in the art. Attachment may be, for example, covalent or reversible, including electrostatic or hydrophobic interactions. In some embodiments, nanoparticles can be added to cells at any point in the process prior to laser irradiation of targeted cells. For example, nanoparticles can be added during the initial cell staining step or after cells have been dispensed into wells.

A method of screening for suitable surface modifications of nanoparticles was devised. Various cell types were loaded with Calcein AM at a final concentration of 1 µM and seeded into multiwell plates. Nanoparticles at a range of concentrations and with different surface modifications were added to different wells. Cells were irradiated with a laser pulse on the LEAP instrument, and cell lysis was measured as the reduction in Calcein AM-positive cells after laser processing. Potency of the different nanoparticle coatings for increasing laser-mediated cell lysis was measured by deriving the EC₅₀ value from nanoparticle concentration versus lysis efficiency dose response curves.

### EXAMPLES

### EXAMPLE 1

### Analysis of Circulating Tumor Cells from Human Blood

In one aspect, human blood from a patient is collected and depleted of erythrocytes using any suitable method, including methods known to those skilled in the art, for instance by lysis of erythrocytes using an ammonium chloride containing buffer. The erythrocyte-depleted blood sample is mixed with a labeled antibody that specifically identifies circulating tumor cells and is detectable by a property of light, such as fluorescence. A phycoerythrin-conjugated anti-EpCAM antibody is an example of such an antibody. Such antibodies can be obtained from a variety of commercially available sources, for example. A mixture of an unlabeled primary antibody and a secondary, labeled antibody directed against the primary antibody may also be used. Examples include a primary mouse anti-EpCAM antibody and a secondary goat-anti mouse, phycoerythrin-labeled antibody. After washing, the cells can be partitioned into a multiwell plate, such as a 384-well plate at a density of 10-100,000 cells per well. Tumor cells may be identified, for example, by imaging the plate to identify tumor cells. If desired, RNAse inhibitor can be added to wells which contain one or more tumor cells, which are then lysed, for example, by irradiating them with a laser pulse sufficient to substantially lyse the targeted cells. The medium may be aspirated. Collected RNA can be analyzed by any suitable method, including for example, by RT-PCR for a select number of genes, by microarray gene expression profiling, by next generation sequencing or any other suitable methodology. In some aspects the generated data can be used, for example, for cancer screening, cancer diagnosis, cancer prognosis, therapy monitoring, therapy choice or in a discovery phase to identify suitable markers for cancer screening, cancer diagnosis, cancer prognosis, therapy monitoring and therapy choice.

### EXAMPLE 2

### Analysis of Lentiviral Transfected Cells

In another aspect a lentiviral library of 100 to 100,000,000 different DNA constructs, such as shRNA, shRNAmir (Thermo Fisher Scientific, Huntsville, AL) or cDNA, is infected into a population of cells. After a period of time, a functional readout in the form of a property of light, for example, a change in the level of a fluorescent reporter protein, such as Green Fluorescent Protein, can be used to identify the cell or cells which contain an infected DNA construct with the desired properties. The desired properties can include, for example, the expression or secretion of a protein, expression of a carbohydrate or lipid, activation or inactivation of a signaling pathway, intra-cellular distribution of a protein, binding properties of a protein, carbohydrate or lipid. If desired, RNAse inhibitor may be added to the wells containing cells to be analyzed. The identified cell or cells can be lysed, for example, by irradiating with a laser pulse sufficient to lyse the targeted cells. Medium may be aspirated, for example and collected. The DNA construct present in the lysed cell may be identified, for example, through PCR, PCR and sequencing, RT-PCR, RT-PCR and sequencing, microarray analysis, next generation sequencing or any other suitable method.

### EXAMPLE 3

### Analysis of Cells Expressing a Protein Variant

In another aspect, a population of cells expressing a DNA library of variants of a protein of interest is analyzed, for example, by imaging, to identify the cell or cells which express the protein variant with the desired properties. The desired properties can include, for example, level of protein expression; intra- and extra-cellular distribution of the protein; folding of the protein; thermal stability of the protein; changes in protein localization after stimulation of the cells with an exogenous agent such as a cytokine, chemokine, interleukin, growth factor, neurotransmitter and lipid. If DNA is to be analyzed, an identified cell may be lysed by irradiating it with a laser pulse sufficient to lyse the targeted cell. Medium may be aspirated and the DNA construct contained within the lysed cell may be identified, for example, by PCR or PCR and subsequent sequencing. If RNA is to be analyzed, RNAse inhibitor can be added to the well in a concentration sufficient to significantly inhibit RNAse activity in the well, and the identified cell may be lysed, for example, by irradiating it with a laser pulse sufficient to lyse the targeted cell. Medium can be aspirated and the nucleic acid construct contained with the lysed cell can be identified, for example, by RT-PCR or RT-PCR and subsequent sequencing, and the like.

### EXAMPLE 4

### Analysis of Tissue Culture Cells

In another aspect, primary cells dissociated from a tissue are cultured in a cell culture plate. A ligand, which may be, for example, a peptide, protein, protein fragment, small molecule, lipid, cannabinoid, aminoalkylindole, eicosanoid, natural extract, fractionated tissue extract, and the like, may be added to the cell culture well containing the cells. Cells which respond to the addition of the ligand can be identified, for example, by a change in the property of light, such as for example, elevation or reduction of intracellular Ca²⁺ measured by a Ca²⁺ responsive dye, such as FLUO4 (Invitrogen); change in cell shape; change in signal from a reporter gene expressed by the cells; or influx into cells of a detectable dye, such as propidium iodide. RNAse inhibitor may be added to the well containing a cell responsive to the addition of the ligand in a concentration sufficient to significantly inhibit RNAse activity in the well. The identified cell may be lysed, for example, by irradiating it with a laser pulse sufficient to lyse the targeted cell. Medium can be collected, for example, by aspiration, and the RNA released into the medium may be profiled, for example, using RT-PCR, quantitative RT-PCR, microarray analysis, next generation sequencing, Nanostring technology, Quantigene assay (Panomics, Fremont, CA), Quantiplex assay (Panomics) or any other suitable methodology. A comparison of the expression data from responsive cells and non-responsive cells can permit, for example, the identification of the mRNA(s) and corresponding protein(s) that are responsible for the responsiveness to the added ligand. Additionally, expression data from the responsive cells can identify the responsive cell type.

### EXAMPLE 5

### Analysis of Circulating Tumor Cells from Human Blood Using "Partitioning" Technique

In another aspect, human blood from a patient is collected, depleted of erythrocytes, and mixed with a labeled antibody that specifically identifies circulating tumor cells and is detectable by a property of light, such as fluorescence. A phycoerythrin-conjugated anti-EpCAM antibody is an example of such an antibody. After washing, the cells are seeded into a multiwell plate, such as a 384-well plate at a density of 100-10,000 per well. The multiwell plate is imaged to identify tumor cells. In some aspects partitioning can be utilized. Partitioning refers to dividing the sample into sub samples and placing the sub samples into bins or separate partitioned containment areas, such as wells of a multiwell plate. In some aspects, the method images substantially the entire area of each bin to determine which bins contain the rare cells. As used herein, "substantially the entire area of each bin" may include for example, greater than, equal to, or any number or range in between 65%, 70%, 75%, 80%, 85%, 90%, 91%, 92%, 93%, 94%, 95%, 96%, 97%, 98%, 99%, 99.5%, 99.6%, 99.7%, 99.8%, 99.9% and 100% of the area of a bin. Due to the partitioning effect of dispensing the blood sample into numerous wells, the concentration of a rare tumor cell will increase in the rare well where it is present. If the number of blood cells per well is 5,000, then the concentration of a single tumor cell will be 1 in 5,000 in the well where it is present and zero in the wells where it is not present. The apparent increase in tumor cell frequency in certain wells where the tumor cells may be found can thus increase the sensitivity of genetic analyses. The well(s) containing one or more tumor cells are lysed, for example using RLT buffer (Qiagen). RNA and/or DNA is extracted and analyzed, for example, by PCR, PCR and sequencing, RT-PCR, RT-PCR and sequencing, quantitative RT-PCR, microarray analysis, next generation sequencing, Nanostring technology, Quantigene assay, Quantiplex assay, or any other suitable method.

### EXAMPLE 6

### Effect of RNAse inhibitor and RNA carrier on the recovery of RNA from lysed cells.

This example describes optimization of RNA recovery from lysed HeLa cells by reagent formulation. A single cell may contain 10-100 pg of total RNA. Lysis of a single cell in a cell culture plate well raised concerns that the small amount of RNA released may be lost due to adsorption onto surfaces or through degradation by RNAses either present in the medium, or released from lysed cells.

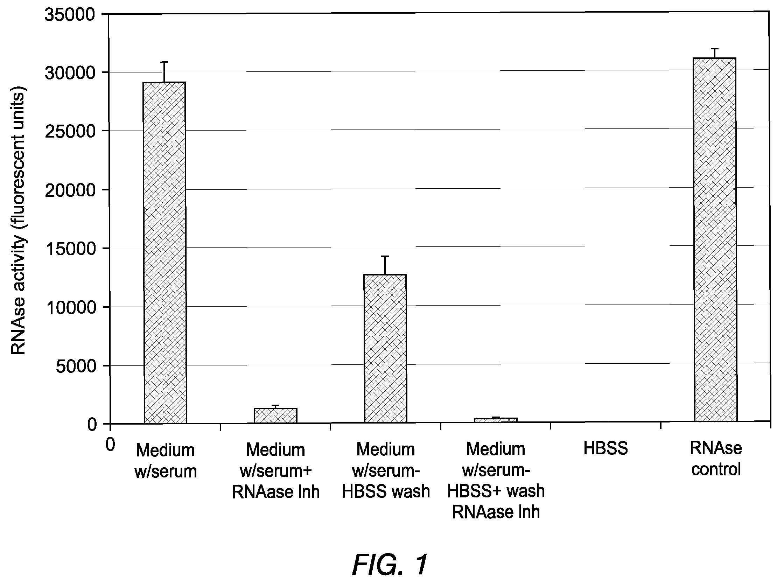

A test for RNAse activity was performed as follows: 384-well C-lect™ plates (Cyntellect, San Diego, CA) were incubated overnight in a cell culture incubator at 37°C with 100 µl of complete medium (MEM - Invitrogen)) with 10% fetal bovine serum (Invitrogen). After incubation, the wells were washed 3 times with Hank's Balanced Salt Solution (HBSS - Invitrogen) using a manual multipipettor. RNAse Alert reagent (Ambion) was mixed according to the manufacturer's recommendations and added to the tissue culture wells. After a 30 minute incubation at 37°C, the samples were read on a Genios plate reader (Tecan). The results are shown in Figure 1. The conditions tested were +/- HBSS wash and +/- addition of an RNAse inhibitor ("RNAse Inh") at a final concentration of 1 unit/µl (ScriptGuard™ - Epicentre Biotechnologies). The results showed that: a) complete medium contains a large amount of RNAse activity, as much as the positive control in the RNAse Alert kit, which is 50x10⁻⁶ units of RNAse A/well; b) addition of a RNAse inhibitor significantly reduced the amount of RNAse activity; c) washing the well with HBSS three times did reduce, but not eliminate, RNAse activity in the well; and d) washing the well with HBSS and adding RNAse inhibitor resulted in the lowest amount of RNAse activity. From this experiment, it was concluded that addition of RNAse inhibitor can facilitate the recovery of RNA from lysed cells in a well.

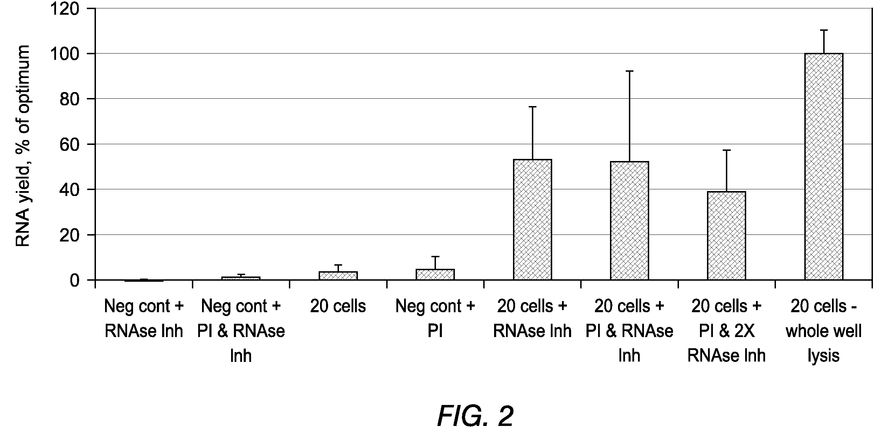

Approximately 200 HeLa cells per well were seeded into a 384-well C-lect plate (Cyntellect). The day after seeding, the cells were stained with CalceinAM (Invitrogen) and wells were washed with HBSS. Twenty HeLa cells were lysed by targeting them with a 532 nm laser pulse with an energy of 10.4 µJ and a laser spot diameter of 17.2 µm using the LEAP instrument (Cyntellect). Half of the medium (HBSS) was collected, from which RNA was extracted using an RNeasy micro column (Qiagen, Valencia, CA). RNA yield was estimated by quantifying GAPDH mRNA as an index of total RNA. GAPDH mRNA was quantified using an absolute quantitative Sybr Green RT-PCR assay on an ABI 7500 real time PCR instrument (Applied Biosystems, Foster City, CA). Reverse transcription was done using the High Capacity cDNA synthesis kit (Applied Biosystems) and Sybr Green PCR was done using the Maxima SYBR Green qPCR mix (Fermentas, Glen Burnie, MD). The following media formulations were tested in various combinations: +/- 1x RNAse inhibitor (RNAse Inh, ScriptGuard, 1U/µl), +/-RNA carrier (PI, polyinosinic acid at 100 ng/well, SIGMA), +/- 2x RNAse inhibitor. As a positive control, all cells in a well were lysed by the addition of RLT (RNeasy kit, Qiagen), containing 100 ng polyinosinic acid, the RNA was purified on an RNeasy micro column and analyzed by RT-PCR as above. Quantities of GAPDH mRNA were normalized against the positive control and are expressed as % of the expected amount. Results are shown in Figure 2. The data showed that: a) lysis of 20 cells in HBSS resulted in a 4.0% yield of GAPDH mRNA; b) addition of polyinosinic acid alone did not improve RNA yield significantly; c) addition of an RNAse inhibitor resulted in a dramatic improvement of RNA yields, 53.3%; and d) increasing the amount of RNAse inhibitor did not improve RNA yields. It was concluded that the addition of an RNAse inhibitor significantly improved RNA yields from lysed cells.

### EXAMPLE 7

### Mutation analysis of a rare tumor cell in human blood cells

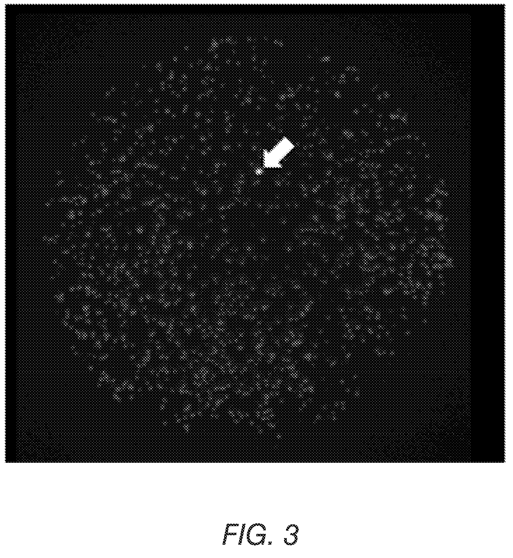

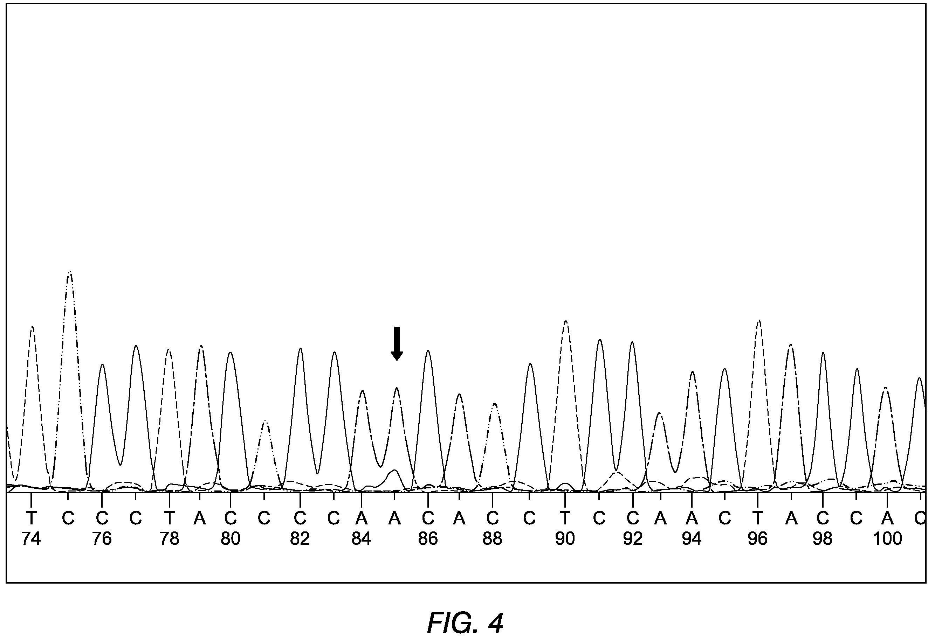

To test the ability to analyze genetic content of rare cells in a relevant model, cells from a human colorectal cell line, SW480, were spiked into human peripheral blood mononuclear cells (PBMCs). SW480 cells are known to harbor a mutation in the Ki-ras gene (McCoy et al., "Characterization of a human colon/lung carcinoma oncogene." Nature. 1983 302(5903):79-81), which is common in colorectal, lung and pancreatic carcinomas (Bos et al., Nature (1987)327: 293-297; Slebos et al., N. Engl. J. Med. (1990) 323: 561-565; and Almoguera et al., Cell (1988) 53: 549-554). To detect the mutation, a fragment of the Ki-ras mRNA containing the mutation was amplified by RT-PCR and the PCR product was sequenced (Eton Bioscience, San Diego, CA). The mutation is a G → T conversion in codon 12 of the Ki-ras gene. To identify SW480 cells, a phycoerythrin-conjugated antibody against EpCAM (eBioscience, San Diego, CA) was used. EpCAM stands for epithelial cellular adhesion molecule. It is specifically expressed by epithelial cells and frequently used to identify and isolate circulating carcinoma cells. SW480 cells were mixed in suspension with fresh PBMCs (AllCells, Hayward, CA) and stained with Calcein AM (Invitrogen) at a concentration of 1µM and the EpCAM antibody at a concentration of 1.25 ng/µl. After 3 washes in HBSS, cells were dispensed into a 384-well C-lect plate (Cyntellect) at a density which resulted in 1600 PBMCs and on average a half of a SW480 cell per well. The plate was imaged on the LEAP instrument (Cyntellect). Wells containing a single SW480 cell were identified and a cocktail of RNAse inhibitor (ScriptGuard - Epicentre Biotechnologies) and polyinosinic acid (Sigma) was added to a final concentration of 1 unit/µl and 100 ng/well respectively in a total volume of 20 µl. Single SW480 cells were lysed by irradiating them with a 532 nm laser pulse, with an energy of 6.9 µJ and a spot diameter of 24 µm. After lysis, half of the medium from the well was aspirated and mixed with RLT from the RNeasy kit (Qiagen). RNA was purified using an RNeasy micro column using the manufacturer's protocol. A fragment spanning the mutation in the Ki-ras mRNA was amplified by RT-PCR using the High Capacity cDNA Reverse Transcription kit (Applied Biosystems) and the Titanium PCR kit (Clontech, Mountain View, CA). The PCR amplicon was amplified in a second round using nested PCR with PCR primers internal to the first primer pair. This step could be used because the abundance of Ki-ras was relatively low in SW480 cells. Nested PCR products were purified using the PCRquick kit (Qiagen) and analyzed by sequencing (Eton Bioscience). Figure 3 shows a single SW480 cell (arrow) in a background of approximately 1,600 PBMCs. The image is originally two color, the SW480 cell is green and PBMCs are red. The SW480 cell was lysed and analyzed by RT-PCR and sequencing. Figure 4 shows the presence of the mutation in the sequence trace derived from analyzing a single SW480 cell lysed by laser-mediated lysis in a background of 1,600 PBMCs. Base 85 is 'A' in the mutated genotype and 'C' in the normal genotype. The sequence trace is generated from the antisense strand of the PCR amplicon, and thus an 'A' corresponds to a 'U' in the mRNA and 'C' corresponds to a 'G' in the Ki-ras mRNA. As the 384-well plate contained in total 1,600 x 384 PBMCs = 614,400 PBMCs, the ability to detect the mutation in one SW480 cell in a well of 1,600 PBMCs translates into the ability to analyze one SW480 cell per 614,400 PBMCs.

### EXAMPLE 8

### Improvement of laser-mediated lysis using gold nanoparticle labeling

For cell lysis to occur upon laser irradiation, the laser pulse must generate a stress or shock wave which mechanically disrupts the cell. The laser energy required to generate this stress or shock wave depends on the absorption of laser light by the cell, medium or substrate that the cell is contacting. Nanoparticle-mediated photolysis has been described as a therapeutic method to eliminate tumor cells from a patient sample (Letfullin et al, 2006). The nanoparticles were used as a means to create optical contrast between targeted tumor cells and non-targeted healthy cells so that predominantly targeted cells were destroyed (Oraevsky et al., 2008). Nanoparticle-conjugated antibodies were used in this example to facilitate the laser-mediated lysis of targeted cells by reducing the laser power required to induce cell lysis. A reduction in laser power reduced the risk of inadvertently causing lysis of adjacent, non-targeted cells, thereby improving the specificity of the analysis of targeted cells.

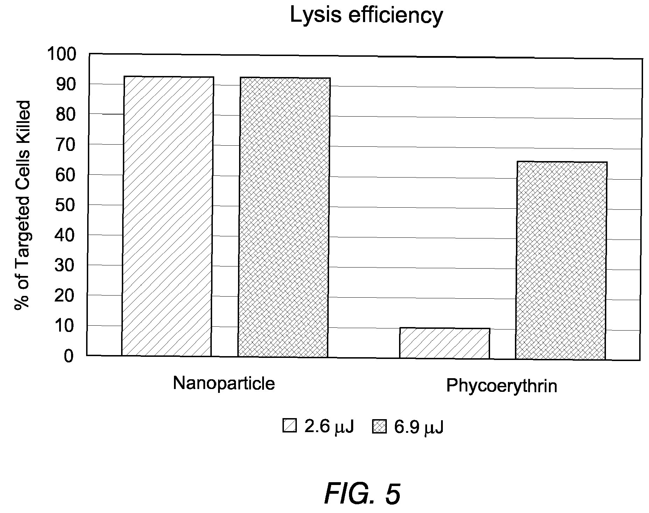

To test the improvement in laser-mediated cell lysis using nanoparticle labeling, SW480 cells in suspension were labeled with an EpCAM antibody (eBioscience) at a concentration of 1.25 ng/µl and with Calcein AM (Invitrogen) at a concentration of 1µM. After 3 washes in HBSS, one set of cells was stained with a secondary gold-labeled antibody (goat-anti mouse, 30 nm - Ted Pella, Redding, CA) at a concentration of 1.15 ng/µl and one set was stained with a phycoerythrin-labeled secondary antibody (eBioscience) at 5 ng/µl. After three washes in HBSS, the stained cells were seeded into a 384-well C-lect plate (Cyntellect) in HBSS. Cell lysis efficiency was measured by quantifying the number of Calcein AM-positive cells, before and after laser processing on LEAP. Figure 5 shows a graph of the laser-mediated cell lysis efficiencies under the different staining and laser power conditions. Lysis efficiency is expressed as % of target cells killed. Using nanoparticle labeling, lysis efficiency was 93% at both laser power settings. In the absence of nanoparticle-labeling (phycoerythrin group in Figure 5), lysis efficiency was 10% at 2.9 µJ and 66% at 6.9 µJ. Thus, nanoparticle labeling improved laser-mediated cell lysis efficiency and reduced the laser power required for efficient cell lysis.

If the nanoparticle labeling is sufficiently strong and specific, specific lysis of the target cell(s) may be achieved by irradiating the entire cell population with an appropriate amount of energy rather than by directing focused energy to the specific target cell(s). There are many examples of photodynamic therapy in which a sensitizer is added to cells, sometimes a gold nanoparticle, such that only the targeted cell(s) absorb(s) a lethal amount of energy whereas adjacent unlabeled non-target cells are not harmed. Examples can be found in Combinatorial treatment of photothermal therapy using gold nanoshells with conventional photodynamic therapy to improve treatment efficacy: An in vitro study, James Chen Yong Kah, et al, Lasers in Surgery and Medicine, Vol 40, Pages 584 - 589, 2008 & Plasmonic photothermal therapy (PPTT) using gold nanoparticles, Xiaohua Huang, et al, Lasers in Medical Science, vol 23, pgs 217-228, 2007. In this aspect, a simpler device and method may be used to practice the technology, one without the steps of imaging, locating the target cell(s), or directing an energy beam specifically to rare target cells.

### EXAMPLE 9

### Gene expression analysis of single tumor cells in a model of circulating tumor cells using nanoparticle-facilitated, laser-mediated lysis

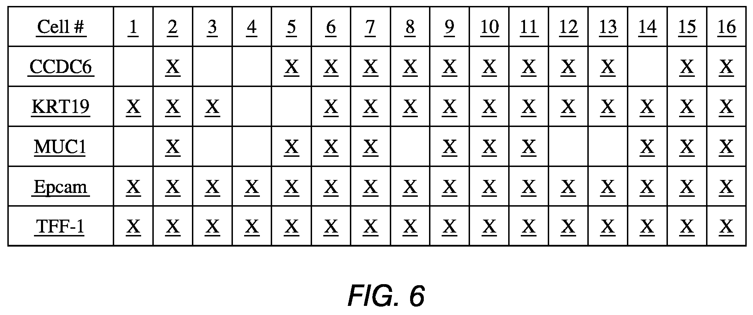

Nanoparticle labeling and subsequent laser-mediated lysis was used to detect the expression of a limited set of genes in single tumor cells spiked into human PBMCs. Human breast cancer cells, MCF-7, were spiked into human PBMCs and stained with a mix of EpCAM antibody (eBioscience) and a phycoerythrin-labeled EpCAM antibody (eBioscience), both at a concentration of 0.625 ng/µl. 100 µl of FcR blocking reagent (Miltenyi, Auburn, CA) was added per 1 ml of cell suspension. After a wash in HBSS with 2% FBS, cells were stained with a secondary gold-labeled antibody (goat-anti mouse, 30 nm, Ted Pella) and washed once again in HBSS with 2% FBS. Finally, the cells were resuspended in HBSS containing 100 µl/ml FcR blocking reagent and 0.27 ng/µl RNase A (Fermentas) to suppress RNA released from cells lysed during the staining process. The cell mix was seeded into a 384-well C-lect plate at 2000 cells/well. The plate was imaged on LEAP. In wells where a MCF-7 cell was detected, ScriptGuard RNase inhibitor (Epicentre biotechnologies) was added to a final concentration of 3 units/µl and polyinosinic acid (Sigma), 100 ng/well. Single MCF-7 cells were lysed by irradiation with a 2.9 µJ laser pulse on the LEAP instrument. One half of the total well volume was aspirated and added to a TargetAmp 1.0 reaction and processed according to the manufacturer's recommendations (Epicentre Biotechnologies) to amplify the mRNAs. The amplified RNA was reverse transcribed into cDNA using the High Capacity cDNA synthesis kit (Applied Biosystems). Quantitative PCR was then performed for the five selected genes using the Maxima SYBR Green qPCR mix (Fermentas) on an ABI 7500 real time PCR instrument (Applied Biosystems). The genes to be analyzed were chosen from the literature based on their relevance to cancer and their expression in MCF-7 cells. The genes were (gene symbols): CCDC6, KRT19, MUC1, EpCAM and TFF-1. Sixteen samples from single, lysed MCF-7 cells were analyzed and 6 negative controls, in which a MCF-7 cell was present in the well, but not lysed, were analyzed. A gene was considered to be expressed if it had a cross over cycle value of <36. For a cell to be classified as positive, at least 3 of the 5 genes had to be expressed. Out of the 16 single tumor cell samples, 15 were classified as positive (Figure 6), corresponding to a 94% successful classification rate. Of the 6 negative samples, none were classified as positive.

### REFERENCES

Allard et al., "Tumor cells circulate in the peripheral blood of all major carcinomas but not in healthy subjects or patients with nonmalignant diseases." Clin Cancer Res. (2004) 10(20):6897-904.
Almoguera et al., "Most human carcinomas of the exocrine pancreas contain mutant c-K-ras genes." Cell (1988) 53: 549-554
Bos et al., "Prevalence of ras gene mutations in human colon cancers" Nature (1987)327: 293-297
Hanania et al., "Automated in situ measurement of cell-specific antibody secretion and laser-mediated purification for rapid cloning of highly-secreting producers." Biotechnol Bioeng. (2005) 30;91(7):872-6.
Letfullin et al.,"Laser-induced explosion of gold nanoparticles: potential role for nanophotothermolysis of cancer." Nanomed. (2006) 1(4):473-80.
Luo et al., "Gene expression profiles of laser-captured adjacent neuronal subtypes" Nat Med. (1999)5(1):117-22
McCoy et al.," Characterization of a human colon/lung carcinoma oncogene."Nature. 1983 302(5903):79-81
Nagrath et al., "Isolation of rare circulating tumour cells in cancer patients by microchip technology." Nature. (2007) 450(7173): 1235-9.
Pastinen et al., " A system for specific, high-throughput genotyping by allele-specific primer extension on microarrays." Genome Res. (2000) 10(7):1031-42.
Pinkel et al., "High resolution analysis of DNA copy number variation using comparative genomic hybridization to microarrays." Nat Genet. (1998) 20(2):207-11
Slebos et al.,"K-ras oncogene activation as a prognostic marker in adenocarcinoma of the lung" N. Engl. J. Med (1990) 323: 561-565.
Smirnov et al., "Global gene expression profiling of circulating tumor cells", Cancer Res. (2005) 65(12):4993-7.
Turkevich et al., "A study of the nucleation and growth processes in the synthesis of colloidal gold", Discuss. Faraday. Soc. 1951, 11, 55-75.
Yan et al., "Dissecting complex epigenetic alterations in breast cancer using CpG island microarrays." Cancer Res. (2001) 1;61(23):8375-80.
Young WS 3rd. "In situ hybridization histochemical detection of neuropeptide mRNA using DNA and RNA probes" Methods Enzymol. (1989) 168:702-10.

U.S. Patents Nos:
5,445,934;
7,378,236;
6,326,489;
7,425,426;
6,514,722;

U.S. Patent Publication. No.:
US2007795857

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation *is* explicitly recited, those skilled in the art will recognize that such recitation should typically be interpreted to mean *at least* the recited number (e.g., the bare recitation of "two recitations," without other modifiers, typically means *at least* two recitations, or *two or more* recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

While various aspects and embodiments have been disclosed herein, other aspects and embodiments will be apparent to those skilled in the art. The various aspects and embodiments disclosed herein are for purposes of illustration and are not intended to be limiting.

## Claims

1. A method for isolating nucleic acids from rare cells within a heterogeneous population of cells, comprising:
(a) partitioning the heterogeneous population of cells into a plurality of bins, such that the concentration of the rare cells relative to non-rare cells is increased within the bins containing the rare cells by a factor of at least X-fold, where X is the number of bins divided by the number of rare cells in the heterogeneous population of cells, wherein the rare cells are present in the heterogeneous population of cells at a concentration of 1 in 1,000 to 1 in 100,000,000;
(b) imaging the entire area of each bin to determine which bins contain the rare cells;
(c) locating the positions of the rare cells within the bins containing the rare cells;
(d) directing an energy beam to the positions of the rare cells, within the bins containing the rare cells, to cause specific lysis of the rare cells without significant lysis of non-rare cells, and release of the nucleic acids from the rare cells into the medium, wherein less than 10% of non-rare cells in the heterogeneous population of cells within the bin are lysed; and
(e) collecting the medium containing released nucleic acids from the bin containing the rare cells that have been lysed, resulting in collection of the nucleic acids from the rare cells.

2. The method of claim 1, wherein X is approximately 10, 30, 100, 300, 1,000, 3,000 10,000, 30,000 or 100,000.

3. The method of claims 1-2, wherein the collecting results in collection of the nucleic acids from the rare cells with up to a Y-fold enrichment of rare cell nucleic acids versus non-rare cell nucleic acids, where Y is the number of unlysed non-rare cells in the bin.

4. The method of claims 1-3, wherein Y is the number of unlysed non-rare cells in the bin and is approximately 10, 30, 100, 300, 1,000, 3,000, 10,000, 30,000, or 100,000.

5. The method of claims 1-4, further comprising the step of contacting the heterogeneous population of cells with an agent that selectively binds to the rare cells, wherein the agent generates a signal detectable as a property of light.

6. The method of claims 1-5, further comprising labeling the cells with a nanoparticle, wherein the nanoparticle improves the efficiency of the energy beam-mediated cell lysis.

7. The method of claims 1-6, further comprising providing an RNAse inhibitor.

8. The method of claims 1-7, further comprising adding an Fc Receptor-blocking reagent.

9. The method of claims 1-8, wherein the starting heterogeneous population of cells has not been enriched to increase concentration of the rare cells relative to non-rare cells.

## Patentansprüche

1. Verfahren zur Isolierung von Nukleinsäuren von seltenen Zellen innerhalb einer heterogenen Population von Zellen, umfassend:
(a) Aufteilen der heterogenen Population von Zellen in eine Vielzahl von Gefäßen, sodass die Konzentration der seltenen Zellen im Verhältnis zu nicht seltenen Zellen innerhalb der Gefäße, die die seltenen Zellen um einen Faktor von mindestens dem X-fachen enthalten, wobei X die Anzahl der Gefäße geteilt durch die Anzahl der seltenen Zellen in der heterogenen Population von Zellen ist, erhöht ist, wobei die seltenen Zellen in der heterogenen Population von Zellen in einer Konzentration von 1 pro 1.000 bis 1 pro 100.000.000 vorliegen.
(b) Abbildung des gesamten Bereich jedes Gefäßes, um zu bestimmen, welche Gefäße die seltenen Zellen enthalten;
(c) Lokalisieren der Positionen der seltenen Zellen innerhalb der Gefäße, die die seltenen Zellen enthalten;
(d) Richten eines Energiestrahls auf die Positionen der seltenen Zellen innerhalb der Gefäße, die die seltenen Zellen enthalten, um eine spezifische Lyse der seltenen Zellen, ohne signifikante Lyse der nicht seltenen Zellen, hervorzurufen und Freisetzung der Nukleinsäuren aus den seltenen Zellen in das Medium, wobei weniger als 10% der nicht seltenen Zellen in der heterogenen Population von Zellen innerhalb des Gefäßes lysiert werden; und
(e) Sammeln des Mediums, welches die freigesetzten Nukleinsäuren enthält, aus den Gefäßen, die die seltenen Zellen, die lysiert wurden, enthalten, wodurch die Nukleinsäuren der seltenen Zellen gesammelt werden.

2. Verfahren nach Anspruch 1, wobei X ungefähr 10, 30, 100, 300, 1.000, 3.000, 10.000, 30.000 oder 100.000 ist.

3. Verfahren nach den Ansprüchen 1 bis 2, wobei die Sammlungsergebnisse beim Sammeln von Nukleinsäuren aus den seltenen Zellen eine bis zur Y-fachen Anreicherung von Nukleinsäuren der seltenen Zellen im Vergleich zu Nukleinsäuren der nicht seltenen Zellen ergibt, wobei Y die Anzahl der nicht lysierten, nicht seltenen Zellen im Gefäß ist.

4. Verfahren nach den Ansprüchen 1 bis 3, wobei Y die Anzahl der nicht lysierten, nicht seltenen Zellen im Gefäß ist und ungefähr 10, 30, 100, 300, 1.000, 3.000, 10.000, 30.000 oder 100.000 ist.

5. Verfahren nach den Ansprüchen 1 bis 4, welches ferner den Schritt des Kontaktierens der heterogenen Population von Zellen mit einem Agens umfasst, das selektiv an die seltenen Zellen bindet, wobei das Agens ein Signal generiert, das als ein Eigenschaft von Licht detektierbar ist.

6. Verfahren nach den Ansprüchen 1 bis 5, welches ferner die Kennzeichnung der Zellen mit einem Nanopartikel umfasst, wobei das Nanopartikel die Effizienz der durch den Energiestrahl vermittelten Zelllyse verbessert.

7. Verfahren nach den Ansprüchen 1 bis 6, welches ferner die Bereitstellung eines RNAse-Inhibitors umfasst.

8. Verfahren nach den Ansprüchen 1 bis 7, welches ferner das Hinzufügen eines Fc-Rezepor-Hemmungsreagenz umfasst.

9. Verfahren nach den Ansprüchen 1 bis 8, wobei die heterogene Ausgangspopulation von Zellen nicht angereichert wurde, um die Konzentration von seltenen Zellen im Verhältnis zu nicht seltenen Zellen zu erhöhen.

## Revendications

1. Procédé d'isolement d'acides nucléiques à partir de cellules rares au sein d'une population hétérogène de cellules, comprenant :
(a) la division de la population hétérogène de cellules dans une pluralité de récipients, de telle façon que la concentration des cellules rares par rapport aux cellules non rares est augmentée au sein des récipients contenant les cellules rares d'un facteur d'au moins X fois, où X est le nombre de récipients divisé par le nombre de cellules rares dans la population hétérogène de cellules, les cellules rares étant présentes dans la population hétérogène de cellules à une concentration de 1 pour 1000 à 1 pour 100 000 000 ;
(b) l'imagerie de la surface entière de chaque récipient pour déterminer quels récipients contiennent les cellules rares ;
(c) la localisation des positions des cellules rares au sein des récipients contenant les cellules rares ;
(d) la direction d'un faisceau d'énergie sur les positions des cellules rares, au sein des récipients contenant les cellules rares, pour provoquer une lyse spécifique des cellules rares sans lyse significative des cellules non rares, et la libération des acides nucléiques à partir des cellules rares dans le milieu, moins de 10 % des cellules non rares dans la population hétérogène de cellules au sein du récipient étant lysées ; et
(e) le recueil du milieu contenant les acides nucléiques libérés à partir du récipient contenant les cellules rares qui ont été lysées, produisant une collection des acides nucléiques provenant des cellules rares.

2. Procédé selon la revendication 1, dans lequel X vaut approximativement 10, 30, 100, 300, 1000, 3000, 10 000, 30 000 ou 100 000.

3. Procédé selon les revendications 1 et 2, dans lequel le recueil produit une collection des acides nucléiques provenant des cellules rares avec un enrichissement de jusqu'à Y fois des acides nucléiques des cellules rares contre les acides nucléiques des cellules non rares, où Y est le nombre de cellules non rares non lysées dans le récipient.

4. Procédé selon les revendications 1 à 3, dans lequel Y est le nombre de cellules non rares non lysées dans le récipient et vaut approximativement 10, 30, 100, 300, 1000, 3000, 10 000, 30 000, ou 100 000.

5. Procédé selon les revendications 1 à 4, comprenant en outre l'étape de mise en contact de la population hétérogène de cellules avec un agent qui se lie sélectivement aux cellules rares, l'agent générant un signal détectable sous la forme d'une propriété de la lumière.

6. Procédé selon les revendications 1 à 5, comprenant en outre le marquage des cellules avec une nanoparticule, la nanoparticule améliorant l'efficacité de la lyse cellulaire médiée par le faisceau d'énergie.

7. Procédé selon les revendications 1 à 6, comprenant en outre la fourniture d'un inhibiteur des ARNases.

8. Procédé selon les revendications 1 à 7, comprenant en outre l'addition d'un réactif bloquant le récepteur Fc.

9. Procédé selon les revendications 1 à 8, dans lequel la population hétérogène de cellules de départ n'a pas été enrichie pour augmenter la concentration des cellules rares par rapport aux cellules non rares.
